(19) Europäisches Patentamt
European Patent Office
Office européen des brevets

(11) **EP 3 608 916 A1**

(12) **EUROPÄISCHE PATENTANMELDUNG**

(43) Veröffentlichungstag:
**12.02.2020 Patentblatt 2020/07**

(51) Int Cl.:
**G16H 40/63** *(2018.01)*

(21) Anmeldenummer: **18188125.1**

(22) Anmeldetag: **09.08.2018**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Justus-Liebig-Universität Gießen
35390 Gießen (DE)**

(72) Erfinder: **Wilbrand, Jan-Falco, Dr.
35392 Gießen (DE)**

(74) Vertreter: **Stumpf, Peter
c/o TransMIT GmbH
Kerkrader Strasse 3
35394 Gießen (DE)**

(54) **VORRICHTUNG UND VERFAHREN ZUR KOPFVERMESSUNG**

(57)   Die Erfindung betrifft eine Vorrichtung 1 zur Kopfvermessung. Diese umfasst wenigstens einen Sender 20, 20a, 20b und einen Empfänger 30, 30a, 30b wobei Sender 20, 20a, 20b und Empfänger 30, 30a, 30b dabei so ausgebildet sind, dass ein vom Sender 20, 20a, 20b an den Empfänger 30, 30a, 30b ausgestrahltes Signal 50 übertragen werden kann. Weiterhin umfasst sie eine Auswerte- und Steuereinheit 40, welche so ausgebildet ist, dass Sender 20, 20a, 20b und Empfänger 30, 30a, 30b Daten mit der Auswerte- und Steuereinheit 40 austauschen können, dass die Auswerte- und Steuereinheit 40 ein Steuersignal an den Sender 20, 20a, 20b senden kann, um zu steuern wann dieser ein Signal 50 an den Empfänger 30, 3a, 30b übertragen kann.

Die Auswerte- und Steuereinheit 40 ist so ausgebildet, dass sie die Laufzeit t des Signals 50 zwischen Sender 20, 20a, 20b und Empfänger 30, 30a, 30b erfassen kann, um aus der Laufzeit t die Länge L einer Messstrecke an einem Kopf bestimmen und so einen Kopf zu vermessen.

Fig.1

# Beschreibung

## Vorrichtung und Verfahren zur Kopfvermessung

Die vorliegende Erfindung betrifft eine Vorrichtung und ein Verfahren zur Vermessung eines Kopfes. Insbesondere soll die Vorrichtung zur Ermittlung der medizinisch wichtigen Kopfparameter wie bspw. der Schädellänge, Schädelbreite und der Länge der transkraniellen Diagonalen dienen.

## Stand der Technik

Seit der Empfehlung der kinderärztlichen Gesellschaften zur ausschließlichen Rückenlagerung von Säuglingen innerhalb des ersten Lebensjahres nehmen kindliche Schädeldeformitäten ("Liegeschädeldeformitäten") massiv an Zahl zu. Aktuell gehen Fachleute von einer Häufigkeit von 1:60 aus. Dies bedeutet, dass eine erste Erfassung der kindlichen Kopfform aktuell sehr häufig notwendig wird, um behandlungsbedürftige Schädeldeformitäten zu erkennen und einer Therapie zuzuführen. Ca. 30% aller Säuglinge zeigen in den ersten 2 Monaten eine passagere Rumpfasymmetrie, die sich auch auf die Schädel- und damit die Kopfform auswirken kann. Die Umsetzung der Empfehlung, Säuglinge auf dem Rücken zu lagern, führte zu einem deutlichen Rückgang der plötzlichen Todesfälle (SIDS). Diese "back to sleep" Kampagne hat nachweislich zu einem höheren Prozentsatz an Lagerungsasymmetrien des kindlichen Kopfes geführt.

Daten zur anthropometrischen Schädelvermessung werden an spezialisierten Zentren erhoben. Dabei unterscheiden sich die Messmethoden der einzelnen Zentren deutlich voneinander. So kommen zum Beispiel manuelle anthropometrische Messungen der Schädellänge, -breite und der Diagonalen mit Maßband und Messzirkel zur Anwendung. Dies erfordert in diesen Messungen geschultes Personal. Alternativ sind Schädelmessungen mit 3D-Photogrammetrie möglich, was einen sehr hohen apparativen und monetären Aufwand erfordert. Ebenfalls sind Schädelmessungen mit Laser, mittels thermoplastischen oder silikonbasierten Kurvenlinealen möglich.

Die relevantesten Kopfparameter sind Schädellänge, Schädelbreite und die Länge der transkraniellen Diagonalen. Die Schädelasymmetrie wird aus der Länge eines Paares der Schädeldiagonalen SDa1 und SDa2 bestimmt.

Ein Paar der Schädeldiagonalen SDa1 und SDa2 sind dabei zwei Diagonalen, die auf den knöchernen Schädel projiziert, durch den Kreuzungspunkt von Längs- und Querdurchmesser des Schädels gehen und jeweils einen festen Winkel $\alpha$ haben, der üblicherweise um etwa 30 Grad vom Längsdurchmesser des Schädels abweicht.

Das Diagonalenverhältnis Diag_Ratio_a ist das Verhältnis eines Paares der Schädeldiagonalen (Sa_Diag A / Sa_Diag B), wobei ein Ergebnis von 1 eine vollkommene Symmetrie darstellen würde.

Die Cranial Vault Asymmetry (CVA) beschreibt den absoluten Längenunterschied eines Paares der Schädeldiagonalen (Sa_Diag A -Sa_Diag B).

Der Cranial Vault Asymmetry Index (CVAI) beschreibt das Verhältnis des absoluten Längenunterschiedes zwischen einem Paar der Schädeldiagonalen geteilt durch die Länge der kürzeren Schädeldiagonalen multipliziert mit 100.

$$CVAI\alpha = \frac{|S\alpha\_\text{Diag}\_A - S\alpha\_\text{Diag}\_B|}{S\alpha\_\text{Diag}\_A} x100$$

Die bisherigen Verfahren zur indirekten anthropometrischen Schädelmessung (z.B. mit thermoplastischen Bändern, Messzirkeln, Schieblehre, Laser 3D Schädel-Photogrammetrie, thermoplastische Bänder) sind oftmals aufwändig, kompliziert und zeitintensiv. Die schnelleren 3D-Verfahren sind sehr teuer und erfordern die Anschaffung umfangreicher 3D-Scanner, die ein komplettes Behandlungszimmer einnehmen. Die hier verwendeten photogrammetrischen Scanner bedürfen einer regelmäßigen umfassenden Kalibrierung, da sie auf 25 (5 x 5) HD-Kameras basieren, die in definierten Winkeln zueinander auf einem Gestänge montiert sind. Vor der Erfassung eines Patienten ist eine etwa 5 - minütige Kalibrierung des Scanners notwendig. Der Aufbau des Scanners führt dazu, dass eine versehentliche Berührung der Kamera-Boxen oder des Gestänges (z.B. durch die Eltern oder Geschwister des Patienten, der photogrammetrisch erfasst werden soll) die Maschine dekalibrieren kann und eine Re-Kalibrierung notwendig wird. Die direkte Anthropometrie (mit Messzirkel und Maßband) stellt eine sehr genaue Methode zur Erfassung von anthropometrischen Messwerten dar, wenn sie von einem erfahrenen und geschulten Untersucher nach einem definierten Messprotokoll durchgeführt wird. Manchmal wird diese Messmethode von Kindern als unangenehm empfunden. Die Messungenauigkeit der direkten anthropometrischen Messungen liegt klinischen Studien zufolge bei etwa 2 mm. Starke Kindsbewegungen könnten jedoch potentiell zu Ungenauigkeiten führen.

## Aufgabe

Aufgabe der vorliegenden Erfindung ist es, die Kopfform insbesondere von Klein(st)kindern einfach, schnell und wenig belastend zu messen. Dazu soll die erfindungsmäße Vorrichtung die Länge L einer Messtrecke bestimmen können, wobei L einen Kopfparameter wie bspw. der Schädellänge, Schädelbreite und der Länge der transkraniellen Diagonalen darstellt.

**Lösung der Aufgabe**

Die Aufgabe wird durch die Vorrichtung mit den Merkmalen des Hauptanspruchs gelöst. Vorteilhafte Weiterbildungen der Erfindung sind in den Unteransprüchen beschrieben.

Die Vorrichtung zur Kopfvermessung 1 umfasst wenigstens einen Sender 20, 20a, 20b und einen Empfänger 30, 30a, 30b.

Sender 20, 20a, 20b und Empfänger 30, 30a, 30b sind so ausgebildet, dass ein vom Sender 20, 20a, 20b an den Empfänger 30, 30a, 30b ausgestrahltes Signal 50 übertragen werden kann. Das Signal 50 kann dabei mechanische oder elektromagnetische Wellen umfassen. Möglich ist dabei beispielhaft die Übertragung von Funkwellen, infrarotem Licht, oder (Ultra-)schall. Dabei lassen sich aus den Signallaufzeiten des Signals 50 die Abstände zwischen je einem Sender 20, 20a, 20b und je einem Empfänger 30, 30a, 30b bestimmen. Um diese sicher bestimmen zu können sollten sowohl Sender 20, 20a, 20b als auch Empfänger 30, 30a, 30b vorzugsweise Mittel zur Zeiterfassung umfassen.

Sender 20, 20a, 20b und Empfänger 30, 30a, 30b sind weiterhin so ausgebildet, dass sie Daten mit einer Auswerte- und Steuereinheit 40 austauschen können. Die Übertragung kann kabelgebunden oder drahtlos erfolgen. Diese Daten umfassen dabei wenigstens die Sende- und Empfangszeiten für das Signal 50.

Weiterhin umfasst die Vorrichtung eine Auswerte- und Steuereinheit 40. Diese ist einerseits so ausgebildet, dass sie ein Steuersignal 60 an den Sender 20, 20a, 20b senden kann, sodass dieser ein Signal 50 an den Empfänger 30, 30a, 30b übertragen kann. Weiterhin ist die Auswerte-und Steuereinheit 40 so ausgebildet, dass sie die Signallaufzeit des Signals 50 zwischen Sender 20, 20a, 20b und Empfänger 30, 30a, 30b erfassen kann.

Aus der Signallaufzeit t des Signals 50 zwischen Sender 20, 20a, 20b und Empfänger 30, 30a, 30b und der Position von Sender 20, 20a, 20b, und Empfänger 30, 30a, 30b kann durch die Steuer-und Auswerteeinheit 40 die Länge L der Messstrecke bestimmt werden. Dabei gilt $L = t \cdot c$, wobei c die Signalgeschwindigkeit, d.h. die Schall- oder die Lichtgeschwindigkeit in Luft ist.

Dies ermöglicht es, einen Kopf zu vermessen. Mit der erfindungsgemäßen Vorrichtung können die Kopflänge, -breite und die transkraniellen Diagonalen sowie weitere Messwerte bestimmt werden. Die Länge L ist dann ein Wert eines Kopfparameters wie bspw. Schädellänge, Schädelbreite oder Länge der transkraniellen Diagonalen. Im einfachsten Fall entspricht die Länge L der Messstrecke also dem Abstand zwischen Sender 20, 20a, 20b und Empfänger 30, 30a, 30b.

In einer optionalen Ausführungsform der Vorrichtung zur Kopfvermessung 1 sind Sender 20, 20a, 20b und Empfänger 30, 30a, 30b so ausgebildet, dass ein Signal 50 in Form elektromagnetischer Strahlung vom Sender 20, 20a, 20b gesendet und vom Empfänger 30, 30a, 30b empfangen werden kann.

Vorteilhafterweise handelt es sich bei der elektromagnetischen Strahlung um infrarotes (IR) Licht einer Wellenlänge zwischen 0,8 μm und 10 μm. Die Verwendung von Strahlung dieser Wellenlänge führt dazu, dass das Kind nicht geblendet wird. Die erfindungsgemäße Vorrichtung ist in der Bereitstellung und Übertragung einfach handhabbar.

In diesem Fall kann es sich bei den Sendern 20, 20a, 20b um IR-Laser handeln und beim Empfänger 30, 30a, 30b um eine IR-Kamera mit einer Photodiode.

In einer optionalen Ausführungsform der Vorrichtung zur Kopfvermessung 1 umfasst diese wenigstens ein Band 10, welches um einen Kopf gelegt und fixiert werden kann. Das Band 10 ist weiterhin so ausgebildet dass, wenigstens ein Sender 20, 20a, 20b oder wenigstens ein Empfänger 30, 30a, 30b an einem Ende der Messstrecke am Band 10 positionierbar ist.

Das Band 10 ist dabei aus einem elastischen Material z.B. Polyamid ausgebildet. Weiterhin kann das Band noch eine Schicht an der Oberfläche aus einem hautfreundlichen und optional waschbaren Material aufweisen. Diese Schicht kann beispielsweise Baumwolle oder Papier umfassen. Die Schicht ist dabei vorzugsweise wechselbar ausgebildet. Damit kann für jede Messung eine saubere Vorrichtung zur Kopfvermessung bereitgestellt werden.

In einer optionalen Ausführungsform der Vorrichtung zur Kopfvermessung 1 umfasst diese noch wenigstens ein Befestigungsmittel 200 am Band 10.

Dieses ist so ausgebildet, dass wenigstens ein wenigstens ein Sender 20, 20a, 20b und/oder Empfänger 30, 30a, 30b an einem Ende der Messstrecke mit dem Befestigungsmittel 200 am Band 10 fixiert werden können.

Weiterhin ist das Befestigungsmittel 200 so ausgebildet, dass wenigstens ein Sender 20, 20a, 20b und/oder wenigstens ein Empfänger 30, 30a, 30b so positioniert werden kann, dass das Signal 50 auf seinem Weg zwischen dem Sender 20, 20a, 20b und dem Empfänger 30, 30a, 30b nicht durch den Kopf behindert wird. Beispielsweise kann das Befestigungsmittel 200 dazu einen Abstandhalter zwischen Band 10 dem Sender 20, 20a, 20b und dem Empfänger 30, 30a, 30b umfassen, welcher dafür sorgt, dass sich Sender 20 und Empfänger 30 oberhalb des höchsten Punktes der Kopfoberfläche befinden. Für Ausführungsformen der Vorrichtung bei denen Sender 20, 20a, 20b und Empfänger 30, 30a, 30b voneinander getrennt angeordnet sind, umfasst die Vorrichtung 1 vorzugsweise für jeden Sender 20, 20a, 20b und für jeden Empfänger 30, 30a, 30b je ein Befestigungsmittel 200.

In einer optionalen Ausführungsform der Vorrichtung zur Kopfvermessung 1 umfasst diese ein Paar aus zwei Empfängern 30a, 30b mit einem festen Abstand D. Dieses Paar aus zwei Empfängern 30a, 30b kann beispielsweise an einer Raumdecke oder alternativ auf einem Stativ in einiger Entfernung zu dem zu vermessenden Objekt angeordnet sein. Als Empfänger sind beispielsweise Infrarotkameras geeignet. Als Sender 20a, 20b dienen dann Infrarot-Lichtquellen. Hierbei sind die Sender 20a, 20b dabei so ausgebildet, *dass* sie an das zu vermessende Objekt z.B. den zu vermessenden Kopf eines Kindes angehalten und der genaue Abstand L zwischen den beiden Sendern gemessen werden kann. Alternativ umfasst die Vorrichtung zur Kopfvermessung 1 ein Paar aus zwei Sendern 20a, 20b mit einem festen Abstand D zueinander. Die Empfänger 20a, 20b sind dabei so ausgebildet, dass sie an das zu vermessende Objekt z.B. den zu vermessenden Kopf eines Kindes angehalten und der genaue Abstand L zwischen den beiden Empfängern gemessen werden kann.

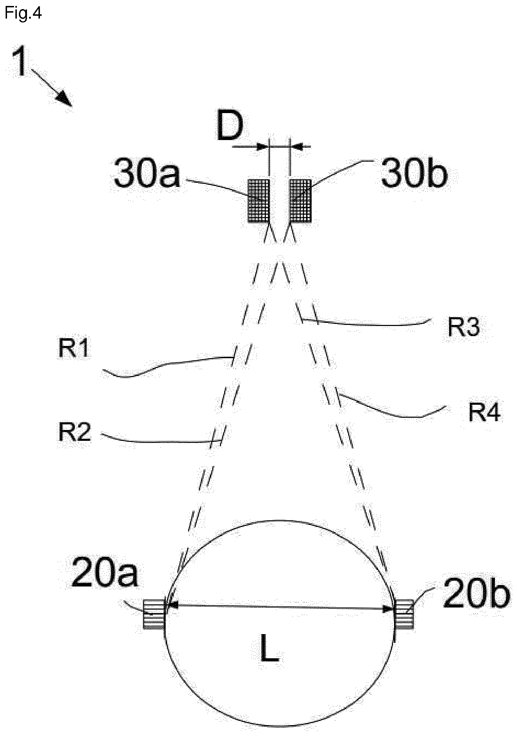

Diese Messung der Länge L kann dann mittels Triangulation aus dem bekannten Abstand D von entweder zwei Sendern 20a, 20b oder von zwei Empfängern 30a, 30b und den Abständen R1, R2, R3, R4 zwischen jedem Sender 20a, 20b und jedem Empfängern 30a, 30b bestimmt werden. Die Abstände R1, R2, R3, und R4. lassen sich dabei aus den Signallaufzeiten t1, t2, t3 und t4 des Signals 50 zwischen den Sendern 20a, 20b und dem Empfängern 30a, 30b bestimmen. Unter Verwendung des Abstandes D kann dann die Länge L mittels Triangulation bestimmt werden. Dies ist in Fig.4 gezeigt.

Die Messung der Signallaufzeiten t1, t2 bzw. t3 und t4 soll vorzugsweise zeitgleich erfolgen. In diesem Fall umfasst die Vorrichtung zur Kopfvermessung 1 zwei Sender 20a, 20b, wobei diese jeweils an einem anderen Ende der Messstrecke positionierbar sind. Dies hat den Vorteil, dass hier nicht der Kopf für zwei Messungen exakt gleich zu den Empfängern 30a, 30b ausgerichtet werden muss.

Die Position der Empfänger 30a, 30b und der Sender 20a, 20b ist dabei grundsätzlich vertauschbar. Das heißt die beiden Sender 20a, 20b können an der Position der beiden Empfänger 30a, 30b angeordnet sein und die beiden Empfänger 30a, 30b an der Position der beiden Sender 20a, 20b. Die Messung ist auch in diesem Fall möglich.

Alternativ kann die Vorrichtung 1 zusätzlich einen Spiegel 100 aufweisen.

In diesem Fall sind Sender 20 und Empfänger 30 gemeinsam an einem ersten Ende der Messstrecke angeordnet. Der Spiegel 100 ist so angeordnet, dass er sich auf der dem Sender 20 und Empfänger 30 entgegengesetzten zweiten Ende der Messstrecke befindet.

In diesem Fall ist die Länge L der Messstrecke einfach der Abstand zwischen dem Paar aus Sender 20 und Empfänger 30 und dem Spiegel 100.

Diese Anordnung ermöglicht die Messung der Signallaufzeit t des Signals 50 vom Sender 20 über den Spiegel 100 zum Empfänger 30. Dabei für die Länge der Messstrecke $$L = \frac{t \cdot c}{2},$$ wobei c die Lichtgeschwindigkeit ist. Wenn der Abstand von Sender 20 und Empfänger 30 sehr gering im Vergleich zur Kopflänge ist, kann der resultierende Messfehler vernachlässigt werden. Sender 20 und Empfänger 30 sind in sehr geringen Abmessungen produzierbar, sodass es möglich ist, diese sehr nahe zueinander zu positionieren. Der Abstand zwischen je einem Sender 20 und einem Empfänger 30 beträgt dabei vorzugsweise weniger als 3 mm. In einer besonderen Ausführungsform der Vorrichtung 1 sind sowohl Sender 20 als auch Empfänger 30 mit einem festen Abstand voneinander angeordnet. Das geschieht idealerweise in einem gemeinsamen Gehäuse.

In einer optionalen Ausführungsform der Vorrichtung zur Kopfvermessung 1 mit einem Spiegel 100 umfasst diese noch mindestens ein Befestigungsmittel 200 am Band 10, welches so ausgebildet ist, dass jeweils ein Paar von Sender 20 und Empfänger 30 und der Spiegel 100 auf eine genau definierte Position am Band 10 zueinander angeordnet und fixiert werden kann. Das Befestigungsmittel 200 ist dabei so ausgebildet, dass der Abstand zwischen jeweils einem Paar von Sender 20 und Empfänger 30 und dem Spiegel 100 der tatsächlichen Messstrecke entspricht. Weiterhin dient das Befestigungsmittel 200 dazu dass sowohl jeweils ein Paar aus Sender 20 und Empfänger 30 soweit vom Kopf entfernt ist, dass das Signal 50 nicht durch den Kopf behindert wird.

**Verfahren:**

Ein erstes Verfahren zur Messung wenigstens eines Kopfparameters mit der erfindungsgemäßen Vorrichtung 1 zur Kopfvermessung umfasst die folgende Schritte:

I. Positionieren je eines Senders 20, 20a, 20b und eines Empfängers 30, 30a, 30b am Kopf an entgegengesetzten Enden der Messstrecke
II. Übertragung eines Signals 50 vom Sender 20, 20a, 20b zum Empfänger 30
III. Messung der Laufzeit t des Signals 50 zwischen Sender 20, 20a, 20b zum Empfänger 30, 30a, 30b durch die Auswerte-und Steuereinheit 40
IV. Bestimmung der Länge L der Messstrecke zwischen Sender 20, 20a, 20b zum Empfänger 30, 30a, 30b aus der Laufzeit t durch die Auswerte-und Steuereinheit 40

Ein alternatives zweites Verfahren zur Messung wenigstens eines Kopfparameters mit der erfindungsgemäßen Vorrichtung 1 zur Kopfvermessung umfasst die folgende Schritte:

I. Positionieren eines Senders 20, 20a, 20b am Kopf an einem ersten Ende der Messstrecke
II. Positionieren eines Empfängers 30, 30a, 30b, sodass ein Signal 50 vom Sender 20, 20a, 20b zum Empfänger 30, 30a, 30b übertragen werden kann

III. Übertragung eines Signals 50 vom Sender 20, 20a, 20b zum Empfänger 30, 30a, 30b

IV. Messung der ersten Laufzeit t1 des Signals 50 zwischen Sender 20, 20a, 20b und Empfänger 30, 30a, 30b durch die Auswerte-und Steuereinheit 40

V. Positionieren eines Senders 20, 20a, 20b am Kopf an einem zweiten Ende der Messstrecke

VI. Übertragung eines Signals 50 vom Sender 20, 20a, 20b zum Empfänger 30, 30a, 30b

VII. Messung der zweiten Laufzeit t2 des Signals 50 zwischen Sender 20, 20a, 20b und Empfänger 30, 30a, 30b durch die Auswerte-und Steuereinheit 40

VIII. Bestimmung der Länge L der Messstrecke zwischen Sender 20, 20a, 20b zum Empfänger 30, 30a, 30b aus den Laufzeiten t1 und t2 durch die Auswerte- und Steuereinheit 40

Ein alternatives drittes Verfahren zur Messung wenigstens eines Kopfparameters mit der erfindungsgemäßen Vorrichtung 1 zur Kopfvermessung umfasst die folgende Schritte:

I. Positionieren eines Empfängers 30, 30a, 30b am Kopf an einem ersten Ende der Messstrecke

II. Positionieren eines Senders 20,20a,20b, sodass ein Signal (50) vom Sender 20,20a,20b zum Empfänger 30, 30a, 30b übertragen werden kann

III. Übertragung eines Signals 50 vom Sender 20, 20a, 20b zum Empfänger 30, 30a, 30b

IV. Messung der ersten Laufzeit t1 des Signals 50 zwischen Sender 20, 20a, 20b und Empfänger 30, 30a, 30b durch die Auswerte-und Steuereinheit 40

V. Positionieren eines Empfängers 30, 30a, 30b am Kopf an einem zweiten Ende der Messstrecke

VI. Übertragung eines Signals 50 vom Sender 20, 20a, 20b zum Empfänger 30, 30a, 30b

VII. Messung der zweiten Laufzeit t2 des Signals 50 zwischen Sender 20, 20a, 20b und Empfänger 30, 30a, 30b durch die Auswerte-und Steuereinheit 40

VIII. Bestimmung der Länge L der Messstrecke zwischen Sender 20, 20a, 20b zum Empfänger 30, 30a, 30b aus den Laufzeiten t1 und t2 durch die Auswerte- und Steuereinheit 40

Ein alternatives viertes Verfahren zur Messung wenigstens eines Kopfparameters mit der erfindungsgemäßen Vorrichtung 1 zur Kopfvermessung umfasst die folgende Schritte:

I. Positionieren von zwei Sendern 20a,20b an jeweils einem Ende der Messstrecke

II. Positionieren von zwei Empfängern 30a, 30b mit einem festen Abstand D zueinander, sodass ein Signal 50 von den Sendern 20a, 20b zum Empfänger 30a, 30b übertragen werden kann

III. Aussenden je eines Signals von jedem Sender 20a, 20b zu jedem Empfänger 30a,30b

IV. Messung der Signallaufzeit t1des Signals 50 zwischen dem Sender 20a und dem Empfänger 30a, der Signallaufzeit t2des Signals 50 zwischen dem Sender 20a und dem Empfänger 30b, der Signallaufzeit t3des Signals 50 zwischen dem Sender 20b und dem Empfänger 30a und der Signallaufzeit t4des Signals 50 zwischen dem Sender 20b und dem Empfänger 30b

V. Bestimmung der Länge L der Messstrecke zwischen den Sendern 20a 20b und den Empfängern 30a, 30b aus den Signallaufzeiten t1, t2, t3, t4 zwischen jedem Sender 20a, 20b zu jedem Empfänger 30a, 30b und der Länge der Strecke D.

Ein alternatives fünftes Verfahren zur Messung wenigstens eines Kopfparameters mit der erfindungsgemäßen Vorrichtung 1 zur Kopfvermessung umfasst die folgende Schritte:

I. Positionieren von zwei Empfängern 30a, 30b an jeweils einem Ende der Messstrecke

II. Positionieren von zwei Sendern 20a,20b mit einem festen Abstand D zueinander, sodass ein Signal 50 von den Sendern 20a, 20b zum Empfänger 30a, 30b übertragen werden kann

III. Aussenden je eines Signals von jedem Sender 20a, 20b zu jedem Empfänger 30a,30b

IV. Messung der Signallaufzeit t1 des Signals 50 zwischen dem Sender 20a und dem Empfänger 30a, der Signallaufzeit t2 des Signals 50 zwischen dem Sender 20a und dem Empfänger 30b, der Signallaufzeit t3 des Signals 50 zwischen dem Sender 20b und dem Empfänger 30a und der Signallaufzeit t4 des Signals 50 zwischen dem Sender 20b und dem Empfänger 30b

V. Bestimmung der Länge L der Messstrecke zwischen den Sendern 20a 20b und den Empfängern 30a, 30b aus den Signallaufzeiten t1, t2, t3, t4 zwischen jedem Sender 20a, 20b zu jedem Empfänger 30a, 30b und der Länge der Strecke D.

Ein alternatives sechstes Verfahren zur Messung wenigstens eines Kopfparameters mit der erfindungsgemäßen Vorrichtung 1 zur Kopfvermessung umfasst die folgende Schritte:

I. Positionieren eines Senders 20 und eines Empfängers 30 am Kopf an einem ersten Ende der Messstrecke

II. Positionieren eines Spiegels 100 am Kopf an einem zweiten Ende der Messstrecke, sodass ein vom Sender 20 emittiertes Signal am Spiegel reflektiert und zum Empfänger 30 übertragen werden kann

III. Übertragung eines Signals 50 vom Sender 20 zum Empfänger 30 über den Spiegel 100

IV. Messung der Laufzeit t des Signals 50 zwischen Sender 20 und Empfänger (30) durch die Auswerte- und Steuereinheit 40

V. Bestimmung der Länge L der Messtrecke zwischen Sender 20 zum Empfänger 30 aus der Laufzeit t durch die Auswerte-und Steuereinheit 40

Die Vorteile der Vorrichtung und des Verfahrens im Vergleich zum Stand der Technik liegen darin, dass kein Hautkontakt erforderlich ist und somit Ankoppelprobleme (Übergangswiderstand) vermieden werden. Durch Wiederholung der Messung kann eine von der Idealform abweichende Form des Umfangs des Kopfes (Schädeldeformation) ermittelt werden.

**Abbildungslegenden und Bezugszeichenliste**

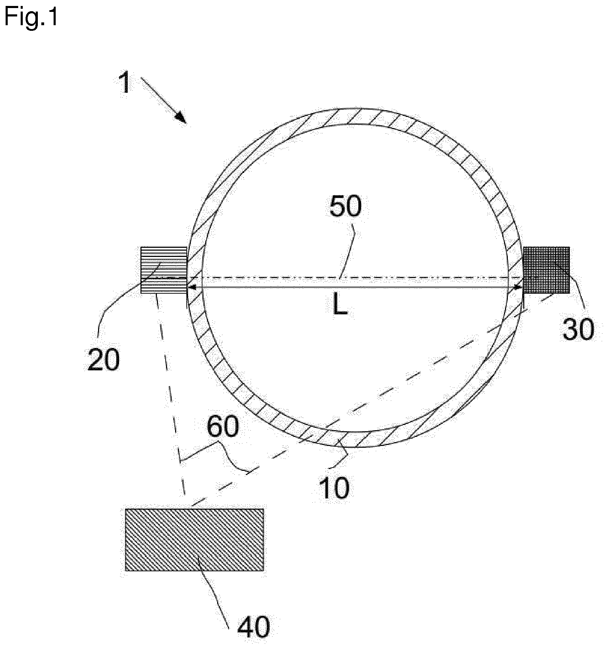

Fig. 1 schematische Darstellung der erfindungsgemäßen Vorrichtung mit einem Band 10 und mit einem Befestigungsmittel 200 (Ansicht von oben).

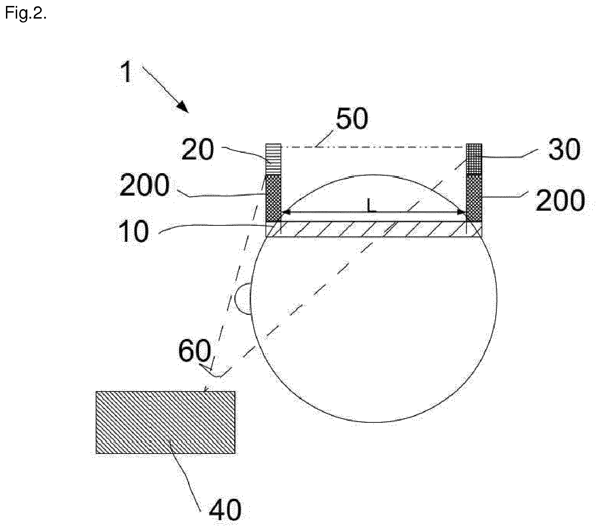

Fig. 2 schematische Darstellung der erfindungsgemäßen Vorrichtung mit einem Band 10 und mit einem Befestigungsmittel 200 (Seitenansicht)

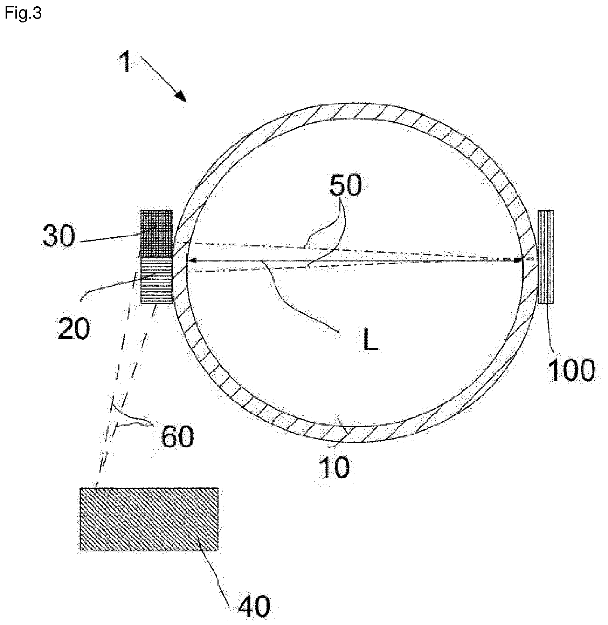

Fig. 3 schematische Darstellung der erfindungsgemäßen Vorrichtung mit einem Spiegel 100 (Ansicht von oben).
Fig. 4 Darstellung der erfindungsgemäßen Vorrichtung mit den gemessenen Wegstrecken R1, R2, R3, R4 für eine Triangulationsmessung zur Bestimmung der Länge L mit zwei Sendern 20a, 20b.

**Bezugszeichen**

1 Vorrichtung zur Kopfvermessung
10 Band
20, 20a, 20b Sender
30, 30a, 30b Empfänger
40 Auswerte- und Steuereinheit
50 Signal
60 Steuersignal
100 Spiegel
200 Befestigungsmittel
L Länge der Messstrecke

**Patentansprüche**

1. Vorrichtung (1) zur Kopfvermessung, durch Bestimmung der Länge L einer Messtrecke an einem Kopf umfassend

• wenigstens einen Sender (20, 20a, 20b) und wenigstens einen Empfänger (30, 30a, 30b) wobei Sender (20, 20a, 20b) und Empfänger (30, 30a, 30b) dabei so ausgebildet sind, dass ein vom Sender (20, 20a, 20b) an den Empfänger (30, 30a, 30b) ausgestrahltes Signal (50) übertragen werden kann, wobei wenigstens ein Sender (20, 20a, 20b) oder wenigstens ein Empfänger (30, 30a, 30b) an einem Ende der Messtrecke an einem Kopf positionierbar ist
• eine Auswerte- und Steuereinheit (40) welche so ausgebildet ist, dass Sender (20, 20a, 20b) und Empfänger (30, 30a, 30b) Daten mit der Auswerte-und Steuereinheit (40) austauschen können.

**dadurch gekennzeichnet, dass** die Auswerte- und Steuereinheit (40) so ausgebildet ist, dass sie die Laufzeit des Signals (50) zwischen dem wenigstens einen Sender (20, 20a, 20b) und dem wenigstens einen Empfänger (30, 30a, 30b) erfassen kann um aus der Signallaufzeit t des Signals (50) die Länge L einer Messstrecke an einem Kopf zu bestimmen.

2. Vorrichtung (1) zur Kopfvermessung gemäß Ansprüche 1 **dadurch gekennzeichnet, dass** Sender (20, 20a, 20b) und Empfänger (20, 20a, 20b) so ausgebildet sind, dass das Signal (50) in Form elektromagnetischer Strahlung übertragen werden kann.

3. Vorrichtung (1) zur Kopfvermessung gemäß Anspruch 1 oder 2 **dadurch gekennzeichnet, dass** diese wenigstens zwei Empfänger (30a, 30b) aufweist, welche einen festen Abstand D zueinander aufweisen, wobei der wenigstens eine Sender (20, 20a, 20b) ein Signal (50) zu jedem der zwei Empfänger (30a, 30b) mit einer Signallaufzeit t1 bzw.t2 übertragen kann.

4. Vorrichtung (1) zur Kopfvermessung gemäß Anspruch 3 **dadurch gekennzeichnet. dass** diese wenigstens zwei Sender (20a, 20b) umfasst, wobei diese jeweils an entgegengesetzten Enden der Messtrecke positionierbar sind und wobei jeder Sender (20a, 20b) ein Signal (50) zu jedem der zwei Empfänger (30a, 30b) übertragen kann.

5. Vorrichtung (1) zur Kopfvermessung gemäß Anspruch 1 oder 2 **dadurch gekennzeichnet, dass** diese wenigstens zwei Sender (20a, 20b) aufweist, welche einen festen Abstand D zueinander aufweisen, wobei jeder Sender (20a, 20b) ein Signal (50) zu wenigstens einem Empfänger (30, 30a, 30b) übertragen kann.

6. Vorrichtung (1) zur Kopfvermessung gemäß Anspruch 5 **dadurch gekennzeichnet, dass** diese wenigstens zwei Empfänger (30a, 30b) umfasst, wobei diese jeweils an entgegengesetzten Enden der Messtrecke positionierbar sind und wobei jeder der zwei Sender (20a, 20b) ein Signal (50) zu jedem der zwei Empfänger (30a, 30b) übertragen kann.

7. Vorrichtung (1) zur Kopfvermessung gemäß einem der vorigen Ansprüche 1 bis 6 **dadurch gekennzeichnet, dass** dieses ein Band (10) umfasst, wobei wenigstens ein Sender (20, 20a, 20b) oder wenigstens ein Empfänger (30, 30a, 30b) an einem Ende der Messtrecke am Band (10) positionierbar ist, wobei das Band (10) um einen Kopf gelegt und fixiert werden kann.

8. Vorrichtung (1) zur Kopfvermessung gemäß Anspruch 7 **dadurch gekennzeichnet, dass** diese am Band (10) wenigstens ein Befestigungsmittel (200) umfasst, wobei wenigstens ein wenigstens ein Sender (20, 20a, 20b) und/oder Empfänger (30, 30a, 30b) über das Befestigungsmittel (200) dem Band (10) fixierbar ist, wobei der wenigstens eine Sender (20, 20a, 20b) und/oder der wenigstens eine Empfänger (30, 30a, 30b) so positioniert werden kann, dass das Signal (50) auf seinem Weg zwischen dem Sender (20, 20a, 20b) und dem Empfänger (30, 30a, 30b) nicht durch den Kopf behindert wird..

9. Vorrichtung (1) zur Kopfvermessung gemäß einem der vorigen Ansprüche 1 bis 8 **dadurch gekennzeichnet, dass** diese weiterhin noch einen Spiegel (100) aufweist, wobei dieser so angeordnet ist, dass er sich auf der dem Sender (20) und Empfänger (30) entgegengesetzten Ende der Messtrecke L befindet.

10. Verfahren zur Messung wenigstens eines Kopfparameters mit einer Vorrichtung (1) zur Kopfvermessung gemäß einem der Ansprüche 1 bis 9 **dadurch gekennzeichnet. dass** es die folgende Schritte umfasst

    I. Positionieren je eines Senders (20, 20a, 20b) und eines Empfängers (30, 30a, 30b) am Kopf an entgegengesetzten Enden der Messtrecke
    II. Übertragung eines Signals (50) vom Sender (20, 20a, 20b) zum Empfänger (30)
    III. Messung der Laufzeit t des Signals (50) zwischen Sender (20, 20a, 20b) zum Empfänger (30, 30a, 30b) durch die Auswerte-und Steuereinheit (40)
    IV. Bestimmung der Länge L der Messtrecke zwischen Sender (20, 20a, 20b) zum Empfänger (30, 30a, 30b) aus der Laufzeit t durch die Auswerte-und Steuereinheit (40)

11. Verfahren zur Messung wenigstens eines Kopfparameters mit einer Vorrichtung (1) zur Kopfvermessung gemäß einem der Ansprüche 1 bis 9 **dadurch gekennzeichnet, dass** es die folgende Schritte umfasst

    I. Positionieren eines Senders (20, 20a, 20b) am Kopf an einem ersten Ende der Messtrecke
    II. Positionieren eines Empfängers (30, 30a, 30b), sodass ein Signal (50) vom Sender (20, 20a, 20b) zum Empfänger (30, 30a, 30b) übertragen werden kann
    III. Übertragung eines Signals (50) vom Sender (20, 20a, 20b) zum Empfänger (30, 30a, 30b)
    IV. Messung der ersten Laufzeit t1 des Signals (50) zwischen Sender (20, 20a, 20b) und Empfänger (30, 30a, 30b) durch die Auswerte-und Steuereinheit (40)
    V. Positionieren eines Senders (20, 20a, 20b) am Kopf an einem zweiten Ende der Messtrecke
    VI. Übertragung eines Signals (50) vom Sender (20, 20a, 20b) zum Empfänger (30, 30a, 30b)
    VII. Messung der zweiten Laufzeit t2 des Signals (50) zwischen Sender (20, 20a, 20b) und Empfänger (30, 30a, 30b) durch die Auswerte-und Steuereinheit (40)
    VIII. Bestimmung der Länge L der Messtrecke zwischen Sender (20, 20a, 20b) zum Empfänger (30, 30a, 30b) aus den Laufzeiten t1 und t2 durch die Auswerte-und Steuereinheit (40)

12. Verfahren zur Messung wenigstens eines Kopfparameters mit einer Vorrichtung (1) zur Kopfvermessung gemäß einem der einem der Ansprüche 1 bis 9 **dadurch gekennzeichnet, dass** es die folgende Schritte umfasst

    I. Positionieren eines Empfängers (30, 30a, 30b) am Kopf an einem ersten Ende der Messtrecke
    II. Positionieren eines Senders (40), sodass ein Signal (50) vom Sender (20, 20a, 20b) zum Empfänger (30, 30a, 30b) übertragen werden kann
    III. Übertragung eines Signals (50) vom Sender (20, 20a, 20b) zum Empfänger (30, 30a, 30b)
    IV. Messung der ersten Laufzeit t1 des Signals (50) zwischen Sender (20, 20a, 20b) und Empfänger (30, 30a, 30b) durch die Auswerte-und Steuereinheit (40)
    V. Positionieren eines Empfängers (30, 30a, 30b) am Kopf an einem zweiten Ende der Messtrecke
    VI. Übertragung eines Signals (50) vom Sender (20, 20a, 20b) zum Empfänger (30, 30a, 30b)
    VII. Messung der zweiten Laufzeit t2 des Signals (50) zwischen Sender (20, 20a, 20b) und Empfänger (30, 30a, 30b) durch die Auswerte-und Steuereinheit (40)
    VIII. Bestimmung der Länge L der Messtrecke zwischen Sender (20) zum Empfänger (30, 30a, 30b) aus den Laufzeiten t1 und t2 durch die Auswerte-und Steuereinheit (40)

13. Verfahren zur Messung wenigstens eines Kopfparameters mit einer Vorrichtung (1) zur Kopfvermessung gemäß einem Ansprüche 1 bis 9, **dadurch gekennzeichnet, dass** es folgende Schritte umfasst

I. Positionieren von zwei Sendern (20a,20b) an jeweils einem Ende der Messstrecke

II. Positionieren von zwei Empfängern (30a, 30b) mit einem festen Abstand D zueinander, sodass ein Signal (50) von den Sendern (20a, 20b) zu jedem Empfänger (30a, 30b) übertragen werden kann

III. Aussenden je eines Signals (50) von jedem Sender (20a, 20b) zu jedem Empfänger (30a,30b)

IV. Messung der Signallaufzeit t1 des Signals (50) zwischen dem Sender (20a) und dem Empfänger (30a), der Signallaufzeit t2 des Signals (50) zwischen dem Sender (20a) und dem Empfänger (30b), der Signallaufzeit t3 des Signals (50) zwischen dem Sender (20b) und dem Empfänger (30a) und der Signallaufzeit t4 des Signals (50) zwischen dem Sender (20b) und dem Empfänger (30b)

V. Bestimmung der Länge L der Messstrecke zwischen den Sendern (20a 20b) und den Empfängern (30a, 30b) aus den Signallaufzeiten t1, t2, t3, t4 zwischen jedem Sender (20a, 20b) zu jedem Empfänger (30a,30b) und der Länge der Strecke D.

14. Verfahren zur Messung wenigstens eines Kopfparameters mit einer Vorrichtung (1) zur Kopfvermessung gemäß einem der Ansprüche 1 bis 9, **dadurch gekennzeichnet, dass** es folgende Schritte umfasst

I. Positionieren von zwei Empfängern (30a, 30b) an jeweils einem Ende der Messstrecke

II. Positionieren von zwei Sendern (20a,20b) mit einem festen Abstand D zueinander, sodass ein Signal (50) von den Sendern (20a, 20b) zu jedem Empfänger (30a, 30b) übertragen werden kann

III. Aussenden je eines Signals von jedem Sender (20a, 20b) zu jedem Empfänger (30a,30b)

IV. Messung der Signallaufzeit t1 des Signals (50) zwischen dem Sender (20a) und dem Empfänger (30a), der Signallaufzeit t2 des Signals (50) zwischen dem Sender (20a) und dem Empfänger (30b), der Signallaufzeit t3 des Signals (50) zwischen dem Sender (20b) und dem Empfänger (30a) und der Signallaufzeit t4 des Signals (50) zwischen dem Sender (20b) und dem Empfänger (30b)

V. Bestimmung der Länge L der Messstrecke zwischen den Sendern (20a 20b) und den Empfängern (30a, 30b) aus den Signallaufzeiten t1, t2, t3, t4 zwischen jedem Sender (20a, 20b) zu jedem Empfänger (30a,30b) und der Länge der Strecke D.

15. Verfahren zur Messung wenigstens eines Kopfparameters mit einer Vorrichtung (1) zur Kopfvermessung gemäß Anspruch 9 **dadurch gekennzeichnet, dass** es die folgenden Schritte umfasst

I. Gemeinsames Positionieren eines Senders (20) und eines Empfängers (30) am Kopf an einem ersten Ende der Messstrecke

II. Positionieren eines Spiegels (100) am Kopf an einem zweiten Ende der Messstrecke, sodass ein vom Sender (20) emittiertes Signal am Spiegel reflektiert und zum Empfänger (30) übertragen werden kann

III. Übertragung eines Signals (50) vom Sender (20) zum Empfänger (30) über den Spiegel (100)

IV. Messung der Laufzeit t des Signals (50) zwischen Sender (20) und Empfänger (30) durch die Auswerte-und Steuereinheit (40)

V. Bestimmung der Länge L der Messstrecke zwischen Sender (20) zum Empfänger (30) aus der Laufzeit t durch die Auswerte-und Steuereinheit (40)

Fig.1

1

50

L

20

30

60

10

40

Fig.2.

Fig.3

Fig.4

**EP 3 608 916 A1**

Europäisches Patentamt
European Patent Office
Office européen des brevets

# EUROPÄISCHER RECHERCHENBERICHT

Nummer der Anmeldung

EP 18 18 8125

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 9 579 045 B2 (SECA AG [CH]) 28. Februar 2017 (2017-02-28) | 1,9,10, 15 | INV. G16H40/63 |
| Y | * Spalte 6 Zeile 28 - Spalte 7 Zeile 48 und Fig. 1 * | 2-8, 12-14 | |
| A | ----- | 11 | |
| Y | US 6 698 289 B1 (LEMCKE SOEREN [DE] ET AL) 2. März 2004 (2004-03-02) | 2-6, 12-14 | |
| A | * Abstrakt, Spalte 2 Zeile 54 - Spalte 3 Zeile 4, Spalte 5 Zeile 11 - 46 und Fig. 1 * | 1,7-11, 15 | |
| Y | ----- US 6 246 898 B1 (VESELY IVAN [US] ET AL) 12. Juni 2001 (2001-06-12) | 7,8 | |
| A | * Fig. 20 * | 1-6,9-15 | |
| X | ----- US 2018/199853 A1 (ABKAI CIAMAK [DE] ET AL) 19. Juli 2018 (2018-07-19) | 1,10 | |
| A | * Paragraphen 18-23 und 28, Fig. 1 und 2 * | 2-9, 11-15 | |
| | | | RECHERCHIERTE SACHGEBIETE (IPC) |
| A | ----- DE 26 07 836 A1 (INST NAT SANTE RECH MED) 9. September 1976 (1976-09-09) * Fig. 1, Seite 2 letzter Absatz und Seite 3 Absatz 1 und 2 * | 1-15 | G16H |
| A | ----- JP S61 104210 A (MATSUSHITA ELECTRIC IND CO LTD) 22. Mai 1986 (1986-05-22) * abstract, par. 1 * ----- | 1-15 | |

Der vorliegende Recherchenbericht wurde für alle Patentansprüche erstellt

| Recherchenort | Abschlußdatum der Recherche | Prüfer |
|---|---|---|
| München | 31. Januar 2019 | Bankwitz, Robert |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

13

## ANHANG ZUM EUROPÄISCHEN RECHERCHENBERICHT
## ÜBER DIE EUROPÄISCHE PATENTANMELDUNG NR.

EP 18 18 8125

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-01-2019

| Im Recherchenbericht angeführtes Patentdokument | Datum der Veröffentlichung | Mitglied(er) der Patentfamilie | Datum der Veröffentlichung |
|---|---|---|---|
| US 9579045 B2 | 28-02-2017 | BR 112015012192 A2 | 11-07-2017 |
| | | CN 104812302 A | 29-07-2015 |
| | | DE 102012220412 B3 | 27-03-2014 |
| | | EP 2925225 A1 | 07-10-2015 |
| | | JP 6236090 B2 | 22-11-2017 |
| | | JP 2016502666 A | 28-01-2016 |
| | | US 2016213283 A1 | 28-07-2016 |
| | | WO 2014082763 A1 | 05-06-2014 |
| US 6698289 B1 | 02-03-2004 | BR 9916441 A | 04-09-2001 |
| | | CN 1324447 A | 28-11-2001 |
| | | CZ 20011165 A3 | 12-09-2001 |
| | | DE 19859202 A1 | 13-07-2000 |
| | | EP 1147369 A1 | 24-10-2001 |
| | | JP 3464786 B2 | 10-11-2003 |
| | | JP 2002533662 A | 08-10-2002 |
| | | KR 100443119 B1 | 04-08-2004 |
| | | US 6698289 B1 | 02-03-2004 |
| | | WO 0037886 A1 | 29-06-2000 |
| US 6246898 B1 | 12-06-2001 | AU 3791099 A | 29-11-1999 |
| | | EP 1076512 A1 | 21-02-2001 |
| | | US 6246898 B1 | 12-06-2001 |
| | | WO 9958055 A1 | 18-11-1999 |
| US 2018199853 A1 | 19-07-2018 | CN 108294755 A | 20-07-2018 |
| | | EP 3349029 A1 | 18-07-2018 |
| | | JP 2018110863 A | 19-07-2018 |
| | | KR 20180083809 A | 23-07-2018 |
| | | US 2018199853 A1 | 19-07-2018 |
| DE 2607836 A1 | 09-09-1976 | DE 2607836 A1 | 09-09-1976 |
| | | FR 2302075 A1 | 24-09-1976 |
| JP S61104210 A | 22-05-1986 | KEINE | |

EPO FORM P0461

Für nähere Einzelheiten zu diesem Anhang : siehe Amtsblatt des Europäischen Patentamts, Nr.12/82